# EUROPEAN PATENT SPECIFICATION

(11) **EP 1 364 030 B1**
(45) Date of publication and mention of the grant of the patent: **15.03.2006**
(21) Application number: 02716745.1
(22) Date of filing: 08.02.2002
(51) Int. Cl.: C12N 15/62

(54) **SUPERSECRETABLE PEPTIDES, PROCESSES FOR THEIR PRODUCTION, AND PARALLEL IMPROVEMENT OF THE SECRETED FORM OF ONE OR MORE OTHER POLYPEPTIDES**
ÜBERSEKRETIERTE PEPTIDE, DEREN HERSTELLUNG, UND GLEICHZEITIGE VERBESSERUNG DER SEKRETIERTEN FORM EINES ODER MEHRERER ANDERER POLYPEPTIDE
PEPTIDES APTES A L'HYPERSECRETION, PROCEDES PERMETTANT LEUR PRODUCTION, ET L'AMELIORATION EN PARALLELE DES FORMES EXPORTEES D'UN OU DE PLUSIEURS POLYPEPTIDES D'INTERET

(30) Priority: 20.02.2001 DE 10108100
(43) Date of publication of application: 26.11.2003
(73) Proprietor: Sanofi-Aventis Deutschland GmbH, 65929 Frankfurt am Main (DE)
(72) Inventor: HABERMANN, Paul, 65817 Eppstein (DE)
(86) International application number: PCT/EP2002/001306
(87) International publication number: WO 2002/066628

(56) References cited:
- US-A- 5 677 172
- THIM L ET AL: "Secretion and processing of insulin precursors in yeast" PROCEEDINGS OF THE NATIONAL ACADEMY OF SCIENCES OF USA, NATIONAL ACADEMY OF SCIENCE. WASHINGTON, US, vol. 83, no. 18, September 1986 (1986-09), pages 6766-6770, XP002100937 ISSN: 0027-8424

### SEQUENCE LISTING

<110> Aventis Pharma Deutschland GmbH
<120> Use of supersecretable peptides in processes for their production and for parallel improvement of the exported forms of one or more other polypeptides of interest
<130> DEAV2001/0007
<140> 10108100.6
   <141> 2001-02-20
<160> 13
<170> PatentIn Ver. 2.1
<210> 1
   <211> 50
   <212> DNA
   <213> Artificial Sequence
<220>
   <223> Description of Artificial Sequence:hir_insfkr
<400> 1
   atccctgagg aataccttca gaagcgattt gttaaccaac acttgtgtgg 50
<210> 2
   <211> 16
   <212> PRT
   <213> Artificial Sequence
<220>
   <223> Description of Artificial Sequence:protein hir_insfkr
<400> 2
   Ile Pro Glu Glu Tyr Leu Gln Lys Arg Phe Val Asn Gln His Leu Cys
   1 5 10 15
<210> 3
   <211> 50
   <212> DNA
   <213> Artificial Sequence
<220>
   <223> Description of Artificial Sequence:hir_insrevkr
<400> 3
   cctcacaagt gttggttaac aaatcgcttc tgaaggtatt cctcagggat 50
<210> 4
   <211> 46
   <212> DNA
   <213> Artificial Sequence
<220>
   <223> Description of Artificial Sequence:hirf1
<400> 4
   tttttttgga tcctttggat aaaagactta cgtatactga ctgcac 46
<210> 5
   <211> 6
   <212> PRT
   <213> Artificial Sequence
<220>
   <223> Description of Artificial Sequence:protein hirf1
<400> 5
   Leu Thr Tyr Thr Asp Cys
   1 5
<210> 6
   <211> 24
   <212> DNA
   <213> Artificial Sequence
<220>
   <223> Description of Artificial Sequence:insnco1rev
<400> 6
   ttttttccat gggtcgacta tcag 24
<210> 7
   <211> 32
   <212> DNA
   <213> Artificial Sequence
<220>
   <223> Description of Artificial Sequence:B_KR_Af1
<400> 7
   cttctacact ccaaagacga aacgcggtat cg 32
<210> 8
   <211> 33
   <212> DNA
   <213> Artificial Sequence
<220>
   <223> Description of Artificial Sequence :B_KR_Arev1
<400> 8
   caacattgtt caacgatacc gcgtttcgtc ttt 33
<210>9
   <211> 33
   <212> DNA
   <213> Artificial Sequence
<220>
   <223> Description of Artificial Sequence :insncorev
<400> 9
   ttttttccat ggtcatgttt gacagcttat cat 33
<210> 10
   <211> 51
   <212> DNA
   <213> Artificial Sequence
<220>
   <223> Description of Artificial Sequence:hir_insfkr
<400> 10
   atccctgagg aataccttca gaagcgattt gtgaaccagc acctgtgcgg c 51
<210> 11
   <211> 51
   <212> DNA
   <213> Artificial Sequence
<220>
   <223> Description of Artificial Sequence:hir_insrevkr
<400> 11
   gccgcacagg tgctggttca caaatcgctt ctgaaggtat tcctcaggga t 51
<210> 12
   <211> 34
   <212> DNA
   <213> Artificial Sequence
<220>
   <223> Description of Artificial Sequence:pichia_H_lf1
<400> 12
   tttttttctc gagaaaagac ttacgtatac tgac 34
<210> 13
   <211> 40
   <212> DNA
   <213> Artificial Sequence
<220>
   <223> Description of Artificial Sequence:pichia_H_1rev2
<400> 13
   tttttggcgc cgaattcact attagttaca gtagttttcc 40

## Description

Use of supersecretable peptides in processes for their production and for parallel improvement of the exported forms of one or more other polypeptides of interest

In view of economic viability, processes for the production of pharmaceutically relevant proteins must lead to biologically active products of the highest possible purity. The expression of such relevant proteins in yeasts is widely used here. The production of proteins such as insulin, GM-CSF (Leukine®) and hirudin (Refludan®) is an example of the successful development of genetic engineering processes which are based on the synthesis of the particular protein or precursors thereof in yeast. Generally, yeasts can directly synthesize particularly hirudins with good yields which are on the gram scale when using Hansenula polymorpha (Weydemann et al., Appl. Microbiol Biotechnol. 44: 377 -385, 1995) or Pichia pastoris (Rosenfeld et al., Protein Expr. Purif :4 , 476 -82, 1996).

EP-A 0 324 712 describes the hirudin derivative (Refludan®) whose N-terminal amino acid is leucine and its constitutive expression in Saccharomyces cerevisiae strain Y79. EP-A 0 347 781 describes a mini-proinsulin and, by way of example, its expression in bakers' yeast. Refludan® and insulin are produced by carrying out two separate expressions.

Surprisingly, we have now found that hirudin derivatives and mini-proinsulin derivatives can be obtained from a common precursor protein by fusing the precursor protein to a signal or leader sequence, which is recognized by yeasts as a secretion signal, via a basic dipeptide, preferably Lys - Arg, and likewise introducing between the N-terminal hirudin derivative and the mini-proinsulin derivative a cleavage site which is recognized by a yeast endoprotease. Here too, preference is given to a basic dipeptide, for example Lys - Arg. After expression, a hirudin derivative extended by Lys - Arg and the mini-proinsulin derivative starting with the first amino acid of the insulin B chain are found in the supernatant Surprisingly, we have found here that the yield of mini-proinsulin markedly improves compared with the yield achievable by direct signal - mini-proinsulin expression, whereas the yield of the hirudin derivative remains nearly the same. Surprisingly, hirudin thus acts as a kind of enhancer peptide with respect to the yield of mini-proinsulin.

Peptides which can act as enhancer proteins are usually those which are relatively small and which are secreted naturally in large amounts over a short period, for example from glandular tissue. Peptides of this type, which include, for example, snake venom or eglin C or TAP (tick anticoagulant peptide), are distinguished by extremely good export compatibility.

Another advantage may result from the hirudin derivative having equal or better pharmaceutical properties compared with hirudin which is already used in pharmaceuticals. In this case, it becomes possible to produce two or even more pharmaceuticals from one and the same fermentation. As a consequence, less fermentation capacity is required. This is directly beneficial to production costs.

However, the production of a plurality of products is optional. The amount needed of Refludan, for example, is less than that of insulin, and this may result in processes in which one of the pharmaceutically interesting substances is discarded.

To improve the yield, it is possible, as suggested in patent application EP-A 0 200 655, to place a short peptide sequence via Lys- Arg N-terminally in front of the hirudin derivative, as a linker to the signal or leader sequence. It is also obvious to the skilled worker that the choice of signal or leader sequence directly affects the yield of the protein of interest. The selection of such a sequence is the subject of further optimizations. The sequence located at the 3' end of the expression cassette, too, directly affects the yield by influencing mRNA stability. Here too, it is obvious to the skilled worker that said sequence can be optimized for each protein of interest to be expressed. This is also true for the choice of a suitable promoter, which can be inducible or constitutively active. The choice of vector system and host system is equally important for the yield. Thus, instead of bakers' yeast which has been used by way of example, it is also possible to use the yeasts Pichia pastoris, Hansenula polymorpha or K. lactis together with vectors or expression cassettes, which have in each case been optimized for the different physiology.

Another advantage of processes allowing secretion into the medium is the simpler protein-chemical workup of the protein of interest. Surprisingly, we have found that mini-proinsulin can be concentrated in the presence of hirudin by filtration through membranes having an exclusion limit for molecules with a molecular weight of greater than 10 kDa. The molecules are found almost exclusively in the retentate. It is obvious to the skilled worker that the development of novel separation techniques and new combinations of process steps always make it possible to improve purification processes. This is directly beneficial to the yield and therefore to production costs.

In Thim et al. 1986: Secretion and processing of insulin precursors in yeast. PNAS, **83**:6766-6770 and US5677172 the yeast mating factor α1 leader sequence and the *S. cerevisiae* hsp150, respectively, are disclosed as heterologous gene expression and secretion enhancing peptides.

The invention thus relates to a DNA-molecule (alternative term: expression cassette) of the form:

Pₓ-Sₓ-Bₙ-(ZR)-transport peptide-(Z₁Z₂)-protein(Y)-(Z₁Z₂)-protein(Yₘ)-T;

wherein the expression cassette codes for a transport peptide linked via a sequence Z₁Z₂to a second protein which in turn is linked via Z₁Z₂ to a protein Y1 which either corresponds to Y or can be different from Y and the transport peptide improves the rate of secretion of Y and/or Yₘ, where:
Pₓ is any promoter DNA sequence selected in such a way that optimal yields of the protein of interest become obtainable;
Sₓ is any DNA which, accordingly, encodes any signal or leader sequence which allows optimal yields;
Bₙ is 1-15 genetically encoded amino acids or a chemical bond;
Z is the codon of an amino acid selected from the group comprising Lys and Arg;
Z₁ is the codon of an amino acid selected from the group comprising Lys and Arg;
Z₂ is the codon of an amino acid selected from the group comprising Lys and Arg;
R is an Arg codon;
transport peptide is a DNA sequence encoding hirudin or a hirudin derivative
protein Y is a DNA sequence encoding any protein which can be produced and secreted by yeast;
protein Yₘ is a DNA sequence encoding any protein which can be produced and secreted by yeast (m = 1-5) or is a chemical bond (m = 0);
T is an untranslated DNA sequence advantageous to the expression.

Another embodiment of the invention is a fusion protein encoded by any of the above-mentioned DNA molecules.

A further embodiment of the invention is a multicopy vector and a plasmid comprising the above-mentioned DNA-molecule.

An additional embodiment of the invention is a host cell comprising the above-mentioned DNA-molecule, or the above-mentioned multicopy vector or the above-mentioned plasmid, as a part of its chromosome, as a part of a mini-chromosome, or extra-chromosomally, wherein preferrentially said host cell is a yeast, in particular selected from the group comprising of S. cerevisiae, K. lactis, H. polymorpha and P. pastoris.

Another embodiment of the invention is a process of fermenting the above-mentioned proteins, in which
(a) the above-mentioned DNA-molecule, the above-mentioned multicopy vector, or the above-mentioned plasmid is expressed in an above-mentioned host cell, and
(b) the expressed proteins are isolated from the supernatant of the cell culture,
wherein in particular after completion of fermentation, the pH is adjusted to 2,5-3,5 in order to precipitate non-desired proteins and the expressed proteins are isolated from the supernatant of the precipitation.

Another embodiment of the invention is the above mentioned process, in which process after separating the fermentation supernatant from the host cells, the host cells are repeatedly cultured in fresh medium, and the released fusion protein is isolated from each supernatant obtained during cultivation.

Another embodiment of the invention is the above mentioned process, wherein a process step for concentrating the expressed protein in the supernatant after precipitation is selected from a group comprising microfiltration, hydrophobic interaction chromatography and ion exchange chromatography.

An additional embodiment of the invention is a process for preparing insulin, in which
(a) proinsulin is expressed as protein (Y) of the above-mentioned expression cassette in the above-mentioned process;
(b) the proinsulin of step (a) is isolated and treated with trypsin and carboxypeptidase B; and
(c) insulin is isolated from the reaction mixture of step (b),
in particular, wherein the transport peptide is hirudin or a hirudin derivative which is destroyed or biologically inactivated after step (a) or (b).

A further embodiment of the invention is a protein wherein the protein is a hirudin-derivative with two basic amino acid residues at its C-terminal end.

Leeches of the type Hirudo have developed, for example, various isoforms of the thrombin inhibitor hirudin. Hirudin has been optimized for pharmaceutical requirements by artificial variation of the molecule, for example exchange of the N-terminal amino acid (e.g. EP 0 324 712). The invention includes the use of hirudin and hirudin variants. Particular embodiments of the invention use one of the natural hirudin isoforms (the natural isoforms are together denoted "hirudin"). A natural isoform is, for example, Val-Val-hirudin or Ile-Thr-hirudin. Other embodiments of the invention use a variant of a natural hirudin isoform. A variant is derived from a natural hirudin isoform but contains, for example, additional amino acids and/or amino acid deletions and/or amino acid exchanges compared with the natural isoform. A hirudin variant may contain alternating peptide segments of natural hirudin isoforms and new amino acids. Hirudin variants are known and are described, for example, in DE 3 430 556. Hirudin variants are commercially available in the form of proteins (Calbiochem Biochemicals, cat. no. 377-853, -950, -960). The term "hirudin derivative" denotes sequences which are at least 40% homologous to natural hirudin.

The expression cassette is preferably introduced into yeasts such as S. cerevisae, K. lactis, H. polymorpha or P. pastoris. Said expression cassette may have one or more copies stably integrated into the particular yeast genome or may be present extrachromosomally on a multicopy vector. It is obvious to the skilled worker that this technique is also applicable to other systems such as animal cell culture or plant cells. This is also a subject of the invention.

The expression system described below serves as an example. It is obvious to the skilled worker that, in order to introduce the expression cassette into said selected system, the appropriate recombinant DNA constructs must be made depending on the type of host system selected. Accordingly, industrial fermentation can be optimized in relation to the selected host/vector system. Accordingly, the examples are nonrestrictive.

### Example 1: Construction of a yeast expression plasmid encoding hirudin (Refludan) - Lys-Arg - mini-proinsulin

Starting materials are the plasmids pK152 (PCT/EP00/08537), pSW3 (EP-A 0 347 781) and the recombinant yeast plasmid derivative coding for bovine interleukin 2 (Price et al. Gene 55, 1987). The yeast plasmid is distinguished by the fact that it carries the α factor leader sequence under the control of the yeast ADH2 promoter. This sequence is followed by the bovine interleukin 2 cDNA sequence which is connected via a Kpnl restriction enzyme recognition site and which contains, after manipulation, an Ncol restriction enzyme recognition site in the untranslated 3' end which is unique in the vector. Thus, the cDNA sequence can readily be removed from the plasmid via Kpnl/Ncol cleavage. Since good expression yields have been reported, it can be assumed that the remaining 3' interleukin 2 sequence (as T) has a stabilizing effect on the mRNA and thus need not be deleted or replaced by a yeast terminator sequence. Plasmid pK152 carries the DNA sequence coding for Leu-hirudin (Refludan) and plasmid pSW3 carries the DNA sequence for mini-proinsulin. The gene sequence which is to encode hirudin - Lys Arg - mini-proinsulin is first prepared by means of PCR technology. For this purpose, 4 primers are prepared with the aid of the Expedite™ DNA synthesis system:
i. hir_insfkr (SEQ ID NO: 1, encoded protein segment: SEQ ID NO: 2)
ii. hir_insrevkr (SEQ ID NO: 3)
iii. hirf1 (SEQ ID NO: 4, encoded protein segment: SEQ ID NO: 5)
iv. insnco1 rev (SEQ ID NO: 6)

Primer hir_insfkr describes the junction between codons for the terminal amino acids of hirudin (59 - 65) and the insulin sequence B1 - B7 via the Lys - Arg linker. Primer hir_insrevkr is 100% complementary thereto. Primer hirf1 codes for the start of the hirudin gene extended to the Kpnl cleavage site as described in EP-A 0 324 712.

Primer insncoirev marks the 3' end of the synthetic mini-proinsulin according to EP-A 0 347 781. Two standard polymerase chain reactions are carried out using the primer pairs hirfl/hir_insrevkr with DNA of plasmid pK152 as template and hir_insfkrlinsncoirev with DNA of plasmid pSW3 as template. The reactions are carried out in 100 µl of PCR buffer with, in each case, 200 nmol of primer, 1 µl of polymerase and 100 ng of vector. Step 1 is a 2-minute incubation at 95°C. This is then followed by 25 cycles of 30" at 95°C, 30" at 55°C and 30" at 72°C. The last cycle is followed by an incubation at 72 °C for 3 minutes, and the reaction is subsequently stopped.

Since the primers hir_insrevkr and hir_insfkr are 100% complementary, the DNA products of the two products overlap according to said sequence so that in a third reaction, using the products of the first two reactions as templates and the primers hirf1 and insncoirev, a DNA fragment is formed, which encodes hirudin and mini-proinsulin separated by Lys - Arg. The PCR fragment is digested by the enzymes Kpnl und Ncol and then, in a T4 ligase reaction, inserted into the pαADH2 vector opened by Kpn1 / Ncol. In analogy to example 7 of EP-A 0 347 781, competent E. coli strain MM294 cells are then transformed with the ligation mixture. Plasmid DNA is then isolated from two clones for characterization by means of DNA sequence analysis. After confirmation of the inserted DNA sequence, DNA of a plasmid preparation is used to transform cells of bakers' yeast strain Y79, according to said example. However, when using the pαADH2 vector, introduction of the vector is followed by selecting for complementation of the trp1-1 mutation, in contrast to said example. For another control, plasmid DNA is reisolated from yeast transformants and analyzed by means of restriction analysis. The expression vector constructed is denoted pADH2Hir_KR_Ins. Expression is carried out according to example 4.

### Example 2: Construction of a yeast expression plasmid encoding hirudin (Refludan) - Lys-Arg - insulin B chain - Lys-Arg - insulin A chain

Patent application EP-A 0 195 691 describes proinsulin derivatives which can contain the dipeptide XY, where X and Y each correspond to either Lys or Arg, as linker between the B and A chains of insulin. The following example describes the preparation of an expression vector for proinsulin derivatives of this kind. A DNA sequence which codes for a proinsulin derivative of the form B chain - Lys-Arg - A chain is selected by way of example and synthesized accordingly.

The synthesis of the gene segment is carried out according to example 1. The oligonucleotide sequences used are hirf1 and insncoirev. The oligonucleotides B_KR_Af1 and B_KR_Arev1 are synthesized de novo.

B_KR_Af1 has the sequence (SEQ ID NO: 7) and B_KR_Arev1 has the sequence (SEQ ID NO: 8)

The part shown in bold type of the two primers depicted indicates the partially overlapping sequence. Both primers pair exactly with the sequence of the mini-proinsulin gene of EP-A 0 347 781, apart from the 6 underlined nucleotides. The underlined part corresponds to codons for Lys and Arg. DNA of the plasmid pADH2Hir_KR_Ins constructed according to example 1 serves as template in the PCR.

As described in example 1, two polymerase chain reactions are carried out using the primer pairs hirf1 / B_KR_Arev and insncoirev / B_KR_Af1. The template in each case is DNA of the plasmid pADH2Hir_KR_Ins constructed in example 1. The products of both reactions serve as template in a third PCR using the primer pair hirf1 and insnco1. The reaction product from PCR 3 is cleaved with Ncol/ Sall and inserted into the opened pαADH2 vector. After sequence and restriction analysis, the correct plasmid is referred to as pADHHirKR_B_KR_A.

### Example 3: Construction of a yeast plasmid coding for hirudin - Lys-Arg - simian proinsulin

Patent application EP-A 489 780 describes a plasmid, pINT90d, which contains cDNA of simian proinsulin (Wetekam et al., Gene 19, p.179-183, 1982). DNA of said plasmid and DNA of plasmid pK152 serve as templates. The primer hirf1 described in example 1 is used and three further primers are synthesized.

Primer insncorev reversely binds to the 3' region of the insulin gene cloned in plNT90d and has the sequence:

The underlined sequence indicates the recognition site for the restriction enzyme Ncol.

Primer hir_insfkr has the sequence:

Here, the nucleotides in bold type indicate the Lys-Arg linker between hirudin and proinsulin.

Primer hir_insrevkr is completely complementary to primer hir_inskr and has the sequence:

Corresponding to example 1, two polymerase chain reactions are carried out. The primer pair hirf1 / hir_insrevkr is reacted with DNA of plasmid pK152 and the primer pair hir_insfkr / insncorev is reacted with DNA of plasmid plNT91d. As described in example 1, the products of both reactions serve as template in a third PCR using the primer pair hirf1 / insncorev. The DNA product of this reaction includes the sequence for hirudin_Lys-Arg_proinsulin. It is subsequently cleaved with the enzymes Ncol and Kpnl and, corresponding to example 1, inserted into the plasmid pαADH2. Acoordingly expression vector for any natural proinsulin derivatives may be constructed.

### Example 4: Expression of the recombinant products

The expression is divided into two phases. Firstly, a preculture is cultivated in yeast minimal medium. The medium has the following composition per 1 l:

| | |
|---|---|
| 6.7 g - | yeast nitrogen base (without amino acids) |
| 5.0 g - | casamino acids (vitamin-free) |
| 0.008% - | adenine |
| 0.008% - | uracil |
| 2% - | glucose |

The main or expression culture is inoculated with an aliquot of the preculture.

The main culture medium contains per liter:

| | |
|---|---|
| 10 g - | yeast extract |
| 20 g - | peptone |
| 0.008% - | adenine |
| 0.008% - | uracil |
| 4% - | glucose |

Using the media described, expression is carried out in a shaken flask in the following way: 0.3 ml of a preculture which has been cultivated overnight is diluted with 80 ml of prewarmed medium and incubated with vigorous shaking at 30°C for approx. 24 h. In each case, 1 ml of the culture produced in this way is then centrifuged, after determining the optical density, and, after removing the cells, the supernatant is lyophilized and analyzed by means of SDS -PAGE. The biologically active hirudin content is determined by carrying out a thrombin inhibition assay.

An alternative fermentation protocol provides for the cells to be removed by filtration or careful centrifugation. While, isolating the protein of interest from the medium, the cells are provided with fresh prewarmed main culture medium containing alcohol and not more than 0.5% glucose as carbon source, and thus fermentation is continued without interruption. This step can be repeated up to 5 times.

### Example 5: Thrombin inhibition test

The hirudin concentration is determined according to the method of Grießbach et al. (Thrombosis Research 37, pp. 347 -350 , 1985). For this purpose, specific amounts of a Refludan standard are included in the measurements in order to establish a calibration curve from which the yield in mg/l can be determined directly.

### Example 6: Cloning and expression of the hirudin - Lys-Arg - mini-proinsulin fusion protein in the P. pastoris system

Invitrogen® sells a cloning and expression kit for preparing recombinant proteins using P. pastoris as host system. For this, a detailed technical protocol regarding preparation and subsequent expression of a P. pastoris system for the production of a desired recombinant protein is provided so that only the construction of the expression vector encoding the desired protein has to be described when following said protocols. The EasySelect™ Pichia expression kit (catalog no. K1740-01) is used.

The pPICZαA vector is part of the kit. Opening the vector by the restriction enzymes Xhol and Sacll makes it possible to append, similar to example 1, a protein of interest to the alpha factor leader sequence and to test for secretion into the supernatant Cloning requires two primers. Primer pichia_H_If1 (SEQ ID NO: 12) has the sequence:

Primer pichia_H_Irev2 (SEQ ID NO: 13) has the sequence:

The template used is DNA of plasmid pADH2Hir_KR_Ins. A standard PCR with both primers produces a DNA product which contains the sequence hirudin -Lys-Arg-mini-proinsulin extended by the Xhol and Sacll integration sites. If the DNA product is cleaved appropriately and the fragment is isolated, said fragment can be inserted into the opened vector DNA in a T4 DNA ligase reaction. In deviation from the manufacturer's protocol, E. coli strain MM294, described in example 1, is transformed with the ligation mixture and recombinant colonies are screened for on zeocine selection plates. Plasmid DNA is reisolated from clones and then characterized by means of restriction and DNA sequence analysis. Using the plasmid constructed in this way, a P. pastoris expression clone for production of the peptides is then prepared, following the manufacturer's instructions.

### Example 7: Purification of mini-proinsulin and hirudin

The purification requires separation of the two proteins at an early stage. After completion of the expression, the medium is analyzed by means of analytical RP-HPLC. In contrast to most other polypeptides found in the supernatant due to either spontaneous lysis of yeast cells or secretion, the two proteins, hirudin and mini-proinsulin, are not precipitated at pH 2.5-3. The culture medium is therefore acidified appropriately and then, after completion of the precipitation, the precipitate and the cells are removed by centrifugation. After centrifugation, the medium is adjusted to pH 3.5-7 and the two components hirudin and mini-proinsulin are separated from one another by means of hydrophobic interaction chromatography, for example by using a chromatography column filled with Diaion HP20® material. Hirudin can then be isolated from the hirudin-containing fractions according to EP-A 0 549 915 and insulin can be isolated from the mini-proinsulin-containing fractions according to EP-A 0 347 781.

### Example 8: Preparation of insulin from mini-proinsulin

At the end of the expression period, the culture medium is adjusted to pH 6.8 and trypsin is then added with stirring so that a final concentration of 4-8 mg per liter is established. After incubation for approx. 4 hours, the fermentation broth treated in this way is adjusted to pH 2.5-3. After 1-6 hours of precipitation, the precipitate is removed. The mono-Arg-insulin formed is then isolated via ion exchange chromatography, by way of example on S - Sepharose® in a buffer of 50 mM lactic acid and 30% isopropanol (pH 3.5). Elution is carried out by means of an NaCl gradient of 0.05--0.5 M salt. The product-containing fractions are diluted 1:1 with H₂O and then ZnCl₂ is added, so that a 0.1 % strength ZnCl₂ solution is formed. Mono-Arg-insulin precipitates at pH 6.8 and by way of example is converted to insulin according to EP-A 0 324 712.

### Example 9: Preparation of insulin from mini-proinsulin after filtration

At the end of the expression period, cells and supernatant components are removed by precipitation at pH 2.5 to 3. Then the medium is concentrated via filtration through membranes having an exclusion limit of 10 kDa. Like the hirudin derivative, mini-proinsulin is found quantitatively in the retentate and can then be processed to insulin according to example 8.

## Claims

1. A DNA-molecule of the form:
Pₓ-Sₓ-Bₙ-(ZR)-transport peptide-(Z₁Z₂)-protein(Y)-(Z₁Z₂)-protein(Yₘ)-T;
wherein the DNA-molecule codes for a transport peptide linked via a sequence Z₁Z₂ to a second protein which in turn is linked via Z₁Z₂ to a protein Y1 which either corresponds to Y or can be different from Y and the transport peptide improves the rate of secretion of Y and/or Yₘ, where
Px is any promoter DNA sequence selected in such a way that optimal yields of the protein of interest become obtainable;
Sx is any DNA which, accordingly, encodes any signal or leader sequence which allows optimal yields;
Bₙ is 1-15 genetically encodable amino acids or a chemical bond;
Z is the codon of an amino acid selected from the group comprising Lys and Arg;
Z₁ is the codon of an amino acid selected from the group comprising Lys and Arg;
Z₂ is the codon of an amino acid selected from the group comprising Lys and Arg;
protein Yₘ is a DNA sequence encoding any protein which can be produced and secreted by yeast (m = 1-5) or is a chemical bond (m = 0);
R is an arginine codon;
transport peptide is a DNA sequence encoding Hirudin or a Hirudin derivative;
protein Y is a DNA sequence encoding any protein which can be produced and secreted by yeast and whose biological activity, when Yₘ is not a chemical bond, is not impaired by a basic dipeptide extension or allows degradation of the extension by carboxypeptidases;
T is an untranslated DNA sequence advantageous to the expression.

2. A DNA-molecule according to claim 1, wherein (Y) is a pharmaceutically relevant protein selected from a group comprising proinsulin and derivatives thereof, interleukins, lymphokines, interferons and factors derived from blood clotting systems.

3. Proteins encoded by any of the DNA molecules according to claims 1 or 2.

4. Multicopy vector comprising the DNA-molecule of any of claims 1 or 2.

5. Plasmid comprising the DNA-molecule of any of claims 1 or 2.

6. Host cell comprising a DNA-molecule of any of claims 1 or 2, a multicopy vector of claim 4 and/or a plasmid of claim 5, as a part of its chromosome, as a part of a mini-chromosome, or extra-chromosomally.

7. Host cell according to claim 6, wherein said host cell is a yeast.

8. Host cell according to claim 7 selected from the group comprising of S. cerevisiae, K. lactis, H. polymorpha and P. pastoris.

9. Process of fermenting proteins according to claim 3, in which
(a) a DNA-molecule of any of claims 1 or 2, a multicopy vector of claim 4, or a plasmid of claim 6 is expressed in a host cell according to any of claims 6 to 8; and
(b) the expressed proteins are isolated from the supernatant of the cell culture.

10. Process according to claim 9, wherein, after completion of fermentation, the pH is adjusted to 2,5-3,5 in order to precipitate non-desired proteins and the expressed proteins are isolated from the supernatant of the precipitation.

11. Process according to claim 10, in which process after separating the fermentation supernatant from the host cells, the host cells are repeatedly cultured in fresh medium, and the released fusion protein is isolated from each supernatant obtained during cultivation.

12. A process according to any of the claims 9 to 11, wherein a process step for concentrating the expressed protein in the supernatant after precipitation is selected from a group comprising microfiltration, hydrophobic interaction chromatography and ion exchange chromatography.

13. Process for the preparation of insulin, in which
(a) proinsulin is expressed as protein (Y) of the expression cassette of claim 1 in a process according to claims 9 or 10;
(b) the proinsulin of step (a) is isolated and treated with trypsin and carboxypeptidase B; and
(c) insulin is isolated from the reaction mixture of step (b).

14. Process according to claim 13, wherein the transport peptide is hirudin or a hirudin derivative which is destroyed or biologically inactivated after step (a) or (b).

15. Protein according to claim 3 wherein the protein is a hirudin-derivative with two basic amino acid residues at its C-terminal end.

## Patentansprüche

1. DNA-Molekül der Form
Pₓ-Sₓ-Bₙ-(ZR)-Transportpeptid-(Z₁Z₂)-Protein(Y)-(Z₁Z₂)-Protein (Yₘ)-T;
wobei das DNA-Molekül für ein Transportpeptid codiert, das über eine Sequenz Z₁Z₂ an ein zweites Protein gebunden ist, das wiederum über Z₁Z₂ an ein Protein Y1 gebunden ist, welches entweder Y entspricht oder von Y verschieden sein kann, und das Transportpeptid die Sekretionsrate von Y und/oder Yₘ verbessert, wobei
Pₓ eine Promotor-DNA-Sequenz ist, die derart ausgewählt ist, dass optimale Ausbeuten des Proteins von Interesse erhältlich werden;
Sₓ eine DNA ist, welche entsprechend für eine Signal- oder Leadersequenz codiert, die optimale Ausbeuten erlaubt;
Bₙ für 1 bis 15 genetisch codierbare Aminosäuren oder eine chemische Bindung steht;
Z das Codon einer Aminosäure, die aus der Lys und Arg umfassenden Gruppe ausgewählt ist, ist;
Z₁ das Codon einer Aminosäure, die aus der Lys und Arg umfassenden Gruppe ausgewählt ist, ist;
Z₂ das Codon einer Aminosäure, die aus der Lys und Arg umfassenden Gruppe ausgewählt ist, ist;
Protein Yₘ eine DNA-Sequenz, die für ein Protein codiert, das durch Hefe produziert und sezerniert werden kann (m=1-5), oder eine chemische Bindung ist (m=0);
R ein Arginincodon ist;
das Transportpeptid eine DNA-Sequenz ist, die für Hirudin oder ein Hirudinderivat codiert;
Protein Y eine DNA-Sequenz ist, die für ein Protein codiert, das durch Hefe produziert und sezerniert werden kann und dessen biologische Aktivität, wenn Yₘ keine chemische Bindung ist, durch eine basische Dipeptidextension nicht beeinträchtigt wird oder einen Abbau der Extension durch Carboxypeptidasen erlaubt;
T eine untranslatierte DNA-Sequenz ist, die für die Expression vorteilhaft ist.

2. DNA-Molekül nach Anspruch 1, wobei (Y) ein pharmazeutisch relevantes Protein ist, das aus einer Gruppe, umfassend Proinsulin und Derivate davon, Interleukine, Lymphokine, Interferone und Faktoren, die aus Blutgerinnungssystem stammen, ausgewählt ist.

3. Proteine, die durch eines der DNA-Moleküle nach Anspruch 1 oder 2 codiert werden.

4. Vektor mit hoher Kopienzahl, der das DNA-Molekül nach Anspruch 1 oder 2 umfasst.

5. Plasmid, das das DNA-Molekül nach Anspruch 1 oder 2 umfasst.

6. Wirtszelle, die ein DNA-Molekül nach Anspruch 1 oder 2, einen Vektor mit hoher Kopienzahl nach Anspruch 4 und/oder ein Plasmid nach Anspruch 5 als Teil ihres Chromosoms, als Teil eines Mini-Chromosoms oder extrachromosomal umfasst.

7. Wirtszelle nach Anspruch 6, wobei die Wirtszelle eine Hefe ist.

8. Wirtszelle nach Anspruch 7, ausgewählt aus der Gruppe umfassend S. cerevisiae, K. lactis, H. polymorpha und P. pastoris.

9. Verfahren zum Fermentieren von Proteinen nach Anspruch 3, in dem
(a) ein DNA-Molekül nach Anspruch 1 oder 2, ein Vektor mit hoher Kopienzahl nach Anspruch 4 oder ein Plasmid nach Anspruch 6 in einer Wirtszelle nach einem der Ansprüche 6 bis 8 exprimiert wird; und
(b) die exprimierten Proteine aus dem Überstand der Zellkultur isoliert werden.

10. Verfahren nach Anspruch 9, wobei nach Beendigung der Fermentation der pH auf 2,5 bis 3,5 eingestellt wird, um nicht erwünschte Proteine zu präzipitieren, und die exprimierten Proteine aus dem Überstand der Präzipitation isoliert werden.

11. Verfahren nach Anspruch 10, wobei in diesem Verfahren nach Abtrennung des Fermentationsüberstandes von den Wirtszellen die Wirtszellen wiederholt in frischem Medium kultiviert werden und das freigesetzte Fusionsprotein aus jedem Überstand, der während der Kultivierung erhalten wird, isoliert wird.

12. Verfahren nach einem der Ansprüche 9 bis 11, wobei ein Verfahrensschritt zur Konzentrierung des exprimierten Proteins im Überstand nach Präzipitation aus der Gruppe, umfassend Mikrofiltration, hydrophobe Wechselwirkungschromatographie und Ionenaustauschchromatographie, ausgewählt wird.

13. Verfahren zur Herstellung von Insulin, in dem
(a) Proinsulin als Protein (Y) der Expressionskassette von Anspruch 1 in einem Verfahren nach Anspruch 9 oder 10 exprimiert wird;
(b) das Proinsulin von Schritt (a) isoliert wird und mit Trypsin und Carboxypeptidase B behandelt wird; und
(c) Insulin aus dem Reaktionsgemisch von Schritt (b) isoliert wird.

14. Verfahren nach Anspruch 13, wobei das Transportpeptid Hirudin oder ein Hirudinderivat, das nach Schritt (a) oder (b) zerstört oder biologisch inaktiviert wird, ist.

15. Protein nach Anspruch 3, wobei das Protein ein Hirudinderivat mit zwei basischen Aminosäureresten an seinem C-terminalen Ende ist.

## Revendications

1. Molécule d'ADN de formule :
Pₓ-Sₓ-Bₙ-(ZR)-peptide de transport-(Z₁Z₂)-protéine(Y)-(Z₁Z₂) -protéine (Yₘ)-T ;
dans laquelle la molécule d'ADN code pour un peptide de transport lié via une séquence Z₁Z₂ à une deuxième protéine qui est liée à son tour via Z₁Z₂ à une protéine Y1 qui correspond à Y ou qui peut être différente de Y et le peptide de transport améliore la vitesse de sécrétion de Y et/ou Yₘ, où
Pₓ est une séquence d'ADN promoteur quelconque sélectionnée de telle manière qu'on peut obtenir des rendements optimaux en protéine à laquelle on s'intéresse ;
Sₓ est un ADN quelconque qui, conformément, code pour une quelconque séquence de signal ou de guidage qui permet des rendements optimaux ;
Bₙ représente 1 à 15 acides aminés pouvant être codés génétiquement ou une liaison chimique ;
Z est le codon d'un acide aminé sélectionné dans le groupe comprenant Lys et Arg ;
Z₁ est le codon d'un acide aminé sélectionné dans le groupe comprenant Lys et Arg ;
Z₂ est le codon d'un acide aminé sélectionné dans le groupe comprenant Lys et Arg ;
protéine Yₘ est une séquence d'ADN codant pour une quelconque protéine qui peut être produite et sécrétée par la levure (m = 1-5) ou représente une liaison chimique (m = 0) ;
R est un codon pour l'arginine ;
le peptide de transport est une séquence d'ADN codant pour l'hirudine ou un dérivé d'hirudine ;
protéine Y est une séquence d'ADN codant pour une quelconque protéine qui peut être produite et sécrétée par la levure et dont l'activité biologique, lorsque Yₘ n'est pas une liaison chimique, n'est pas affectée par une extension par un dipeptide basique ou permet la dégradation de l'extension par des carboxypeptidases ;
T est une séquence d'ADN non translatée avantageuse pour l'expression.

2. Molécule d'ADN selon la revendication 1, dans laquelle (Y) est une protéine présentant une importance pharmaceutique choisie dans le groupe comprenant la pro-insuline et les dérivés de celle-ci, les interleukines, les lymphokines, les interférons et les facteurs dérivés de systèmes de coagulation du sang.

3. Protéines codées par l'une quelconque des molécules d'ADN selon les revendications 1 ou 2.

4. Vecteur multicopies comprenant la molécule d'ADN selon l'une quelconque des revendications 1 ou 2.

5. Plasmide comprenant la molécule d'ADN selon l'une quelconque des revendications 1 ou 2.

6. Cellule hôte comprenant une molécule d'ADN selon l'une quelconque des revendications 1 ou 2, un vecteur multicopies selon la revendication 4 et/ou un plasmide selon la revendication 5, faisant partie de son chromosome, faisant partie d'un mini-chromosome ou de manière extra-chromosomique.

7. Cellules hôte selon la revendication 6, ladite cellule hôte étant une levure.

8. Cellule hôte selon la revendication 7 choisie dans le groupe comprenant S. cerevisiae, K. lactis, H. polymorpha et P. pastoris.

9. Procédé de fermentation de protéines selon la revendication 3, dans lequel :
(a) une molécule d'ADN selon l'une quelconque des revendications 1 ou 2, un vecteur multicopies selon la revendication 4 ou un plasmide selon la revendication 6 est exprimé dans une cellule hôte selon l'une quelconque des revendications 6 à 8; et
(b) les protéines exprimées sont isolées de la phase surnageante de la culture cellulaire.

10. Procédé selon la revendication 9, dans lequel, après la fin de la fermentation, le pH est ajusté à 2,5-3,5 afin de précipiter les protéines non souhaitées et les protéines exprimées sont isolées de la phase surnageante de la précipitation.

11. Procédé selon la revendication 10, dans lequel, après séparation de la phase surnageante de la fermentation des cellules hôtes, les cellules hôtes sont cultivées de manière répétitive dans du milieu frais et la protéine fusionnée libérée est isolée de chaque phase surnageante pendant la culture.

12. Procédé selon l'une quelconque des revendications 9 à 11, dans lequel une étape de procédé pour concentrer la protéine exprimée dans la phase surnageante après la précipitation est choisie dans un groupe comprenant la microfiltration, la chromatographie par interaction hydrophobe et la chromatographie par échange ionique.

13. Procédé de préparation d'insuline, dans lequel :
(a) la pro-insuline est exprimée sous forme d'une protéine (Y) de la cassette d'expression selon la revendication 1 dans un procédé selon les revendications 9 ou 10 ;
(b) la pro-insuline de l'étape (a) est isolée et traitée avec de la trypsine et la carboxypeptidase B ; et
(c) l'insuline est isolée du mélange réactionnel de l'étape (b).

14. Procédé selon la revendication 13, dans lequel le peptide de transport est l'hirudine ou un dérivé d'hirudine qui est détruit ou inactivé biologiquement après l'étape (a) ou (b).

15. Protéine selon la revendication 3, dans laquelle la protéine est un dérivé d'hirudine avec deux résidus d'acides aminés basiques en sa terminaison C.
